# EUROPEAN PATENT SPECIFICATION

(11) **EP 1 387 167 B1**
(45) Date of publication and mention of the grant of the patent: **07.05.2008**
(21) Application number: 02291879.1
(22) Date of filing: 24.07.2002
(51) Int. Cl.: G01N 33/50, C07K 14/705

(54) **Method for identifying biologically active compounds with anti-asthmatic and/or anti-allergic properties**
Verfahren um biologische aktive Verbindungen zu identifizieren, die anti-asthmatische und/oder anti-allergische Eigenschaften haben
Méthode pour isoler des composés biologiques actifs ayant des propriétés anti-asthmatiques et/ou anti-allergiques

(43) Date of publication of application: 04.02.2004
(73) Proprietor: CENTRE NATIONAL DE LA RECHERCHE SCIENTIFIQUE (CNRS), 75794 Paris Cedex 16 (FR)
(72) Inventor: Ryffel, Bernhard, 45560 Saint Denis en Val (FR); Couillin, Isabelle, 41500 Avaray (FR)
(74) Representative: Breese, Pierre

(56) References cited:
- WO-A-01/36488
- WO-A-01/55386
- WO-A-01/74375
- WO-A-99/52549
- WO-A-99/66947
- LIEN E ET AL: "Toll - like receptor 2 functions as a pattern recognition receptor for diverse bacterial products" JOURNAL OF BIOLOGICAL CHEMISTRY, AMERICAN SOCIETY OF BIOLOGICAL CHEMISTS, BALTIMORE, MD, US, vol. 274, no. 47, 19 November 1999 (1999-11-19), pages 33419-33425, XP002161197 ISSN: 0021-9258
- TAKEUCHI OSAMU ET AL: "Cutting Edge: Role of toll-like receptor 1 in mediating immune response to microbial lipoproteins." JOURNAL OF IMMUNOLOGY, vol. 169, no. 1, 1 July 2002 (2002-07-01), pages 10-14, XP002228542 July 1, 2002 ISSN: 0022-1767
- HALLMAN MIKKO ET AL: "Toll-like receptors as sensors of pathogens." PEDIATRIC RESEARCH, vol. 50, no. 3, September 2001 (2001-09), pages 315-321, XP002228543 ISSN: 0031-3998
- FLO T H; HALAAS O; LIEN E; RYAN L; TETI G; GOLENBOCK D T; SUNDAN A; ESPEVIK T: "Human toll - like receptor 2 mediates monocyte activation by Listeria monocytogenes, but not by group B streptococci or lipopolysaccharide" JOURNAL OF IMMUNOLOGY, vol. 164, no. 4, 15 February 2000 (2000-02-15), pages 2064-2069, XP002161196 USA ISSN: 0022-1767
- AKIRA SHIZUO; TAKEDA KIYOSHI; KAISHO TSUNEYASU: "Toll-like receptors: Critical proteins linking innate and acquired immunity" NATURE IMMUNOLOGY, vol. 2, no. 8, August 2001 (2001-08), pages 675-680, XP002974836 ISSN: 1529-2908

## Description

The present invention concerns new methods for identifying biologically active compounds with anti-asthmatic, anti-allergic and/or immunosuppressive properties.

Several factors may affect allergic asthma. Allergic asthma is characterised by increased antigen specific serum IgE level and eosinophilic inflammation of the airway, which are accompanied by increased airway reactivity. The cytokines IL-4 (for interleukin-4), IL-5 (for interleukin-5) and IL-13 (for interleukin-13) appear to play a key role in the pathogenesis of allergic diseases. Antigen/allergen induces in sensitised individuals cross-linking of IgE on mast cells followed by the release of several preformed bioactive molecules such as, histamine, tryptase, serotonine eosinophil chemotactic factor (ECF), resulting in acute bronchoconstriction, mucus secretion, vasodilatation and by the release of neoformed molecules like leukotrienes, prostaglandins and several cytokines (IL-4, IL-5, tumor necrosis factor α for TNF-α) resulting in lung inflammation.
The incidence of asthma increased in the last twenty years and the need of more specific therapies is real.

Toll receptors were first described in drosophila where the Toll protein controls dorsoventral patterning during the embryonic development of Drosophila (Hashimoto et al., 1988, Cell, vol. 52, pages 269-279; Belvin and Anderson, 1996, Annu. Rev. Cell Dev. Biol., vol. 12, pages 393-416) and is required for antifungal immune response in adult fly (Lemaitre and al., 1996, Cell, vol. 86, pages 973-983). Toll is a type I transmembrane receptor with an extracellular domain containing leucine-rich repeat (LRR) and a cytoplasmic domain similar to that of mammalian interleukin-1 receptor.
Mammalian homologues of Toll, designated as Toll-like receptors (TLRs) have been identified. The mammalian TLRs are structurally conserved and homologous to the Drosophila Toll system (Medzhitov and al., 1997, Nature, vol. 388, pages 394-397). Ten members of the TLR family have been reported to date and these members are called TLR1 to TLR10 (Underhill and Ozinski, 2002, Curr. Opin. Immunol., vol. 14, pages 103-110). The TLR family members are known to activate the transcription factor NFₖB via an adapter protein MyD88 and a serine/threonine kinase IRAK (Medzhitov et al., 1998, Mol. Cell., vol. 2, pages 253-258 ; Muzio et al., 1998, J. Exp. Med., vol. 187, pages 2097-2101 ; Kawai et al., 1999, Immunity, vol. 11, pages 115-122). Moreover, mammalian TLRs play an essential role in innate immunity by recognising conserved pathogen-associated molecular patterns and initiating the activation of NF-κB and other signalling pathways through the adapter protein MyD88 (Schnare et al., 2001, Nature Immunology, vol. 2, pages 947-950).

Studies showed that, among the TLR family members, TLR2 is implicated in the recognition of various bacterial cell wall components. Takeuchi et al. (1999, Immunity, vol. 11, pages 443-451) demonstrated that TLR2 plays a major role in Gram-positive bacterial recognition since TLR2 is essential for responsiveness to bacterial peptidoglycan (PGN) which is a component essential for Gram-positive bacterial recognition. Thus, TLR2 mediates cellular responses to a wide variety of infectious pathogens and their products. These include yeast cell walls, whole bacteria, mycobacterial lipoarabinomannan, whole Gram-positive bacteria, peptidoglycan (PGN), *Treponema* glycolipid and *Trypanosoma cruzi* glycophosphatidylinositol anchor (for review, see Akira et al., 2001, Nature Immunology, vol. 2, pages 675-680).

In addition, TLR2 mediates the responses to lipoproteins which are secreted antigens derived from *M*. *tuberculosis, Borrelia burgdorfei, Treponema pallidium* and *Mycoplasma fermentans* (for review, see Akira et al., 2001, Nature Immunology, vol. 2, pages 675-680). Moreover, Werts et al. (2001, Nature Immunology, vol. 2, pages 346-352) disclosed that TLR2 is required for innate immune responses to leptospiral lipopolysaccharides (LPS), *Leptospira interrogans* belonging to the Spirochaetales order which constitutes one phylum of eubacteria.

It has also been demonstrated that TLR2 must dimerize with other TLRs as TLR1 or TLR6 or others TLRs to detect ligands and induce signalling (Ozinsky et al., 2000, PNAS, vol. 97, pages 13766-13771; Takeuchi et al., 2002, J. Immunol., vol. 169, pages 10-14).

The following documents
LIEN E ET AL: "Toll - like receptor 2 functions as a pattern recognition receptor for diverse bacterial products" JOURNAL OF BIOLOGICAL CHEMISTRY, AMERICAN SOCIETY OF BIOLOGICAL CHEMISTS, BALTIMORE, MD, US, vol. 274, no. 47, 19 November 1999 (1999-11-19), pages 33419-33425, XP002161197 ISSN: 0021-9258, WO 01 /36488 A,
TAKEUCHI OSAMU ET AL: "Cutting Edge: Role of toll-like receptor 1 in mediating immune response to microbial lipoproteins." JOURNAL OF IMMUNOLOGY, vol. 169, no. 1, 1 July 2002 (2002-07-01), pages 10-14, XP002228542 July 1, 2002 ISSN: 0022-1767, and FLO T H; HALAAS O; LIEN E; RYAN L; TETI G; GOLENBOCK D T; SUNDAN A; ESPEVIK T: "Human toll - like receptor 2 mediates monocyte activation by Listeria monocytogenes, but not by group B streptococci or lipopolysaccharide" JOURNAL OF IMMUNOLOGY, vol. 164, no. 4, 15 February 2000 (2000-02-15), pages 2064-2069, XP002161196 USA ISSN: 0022-1767
disclose testing various compounds such as bacterial ones for their ability to alter (e.g. increase) the basal activity of TLR2 in cells.
WO 01/55386 A discloses screening methods for modulators of TLR9 useful e.g. against asthma.
WO 99/66947 A provides methods and compounds for the treatment of allergic and other immune disorders associated with overproduction of Th2 type cytokines by antigen specific T cells.
WO 01/74375 A provides compositions and methods for inhibiting immune responses associated with autoimmune diseases and allergic responses.
WO 99/52549 A discloses vaccine compositions to protect against infections, allergy and cancer.

The inventors asked whether the signalling receptor of innate immunity, TLR2, has a regulatory role in an experimental model of allergic asthma. Using TLR2 deficient mice, the inventors found a dramatic increase of bronchial hyperreactivity (BHR) upon ovalbumin challenge in immunised mice. Increased BHR was associated with a 3-fold increase in eosinophils in the BAL (Bronchoalveolar lavage) fluid and in the lung tissue. Conversely, local administration of lipoprotein (LP), a natural agonist of TLR2, inhibited BHR and eosinophilia in the lungs. The data demonstrate for the first time that TLR2 plays a critical role in the control of allergen induced asthma, and activation of TLR2 prevents allergic asthma. Therefore, TLR2 represents a drug target of novel therapeutics in bronchial asthma and allergic disorders in general.

The present invention concerns a method for identifying a biologically active compound with anti-asthmatic and/or anti-allergic properties wherein it comprises the following steps:
a) Contacting a TLR2 with said biologically active compound, and
b) Determining if said biologically active compound increases the basal activity of that said TLR2.

By biologically active compound, the invention gives particular consideration to natural or synthetic chemical compounds such as, for example, but not restricted to proteins, polypeptides, peptides, lipoproteins, polysaccharides, etc.... The validation of the biological activity of said compound can be done using a sensitised animal model in which the bronchial hyperreactivity (BHR), the amount of eosinophils in the BAL (Bronchoalveolar lavage) fluid and in the lung tissue are tested and compared before and after the administration of said compound.

In a first embodiment, the step (a) in the method object of the present invention comprises the following steps:
i) Culturing cells which express functional TLR2, and
ii) Incubating said cells with said biologically active compound.

Advantageously, the cells cultured in the step (i) of the method object the invention are cells which over-express TLR2. Any cell, which can over-express TLR2, can be used in the scope of the invention. Said cells are preferably mammalian cells, said mammalian cells are chosen in the group constituted by human HEK293 and murine BAF3 cells.
A molecule of encoding nucleic acid for TLR2 or a vector as disclosed above can be used to transform the cells, which over-express TLR2.
The molecule of encoding nucleic acid for TLR2 is advantageously a molecule encoding for a mammalian TLR2 and, more preferably, a molecule encoding for a human TLR2. The sequences of these molecules can be obtained in Genbank under the numbers (NM_003264 for human and NM_011905 for mouse).
Due to the degeneracy of nucleotide coding sequences, other DNA sequences, which encode substantially the same amino acid sequence as TLR2 gene may be used in the practice of the present invention. These include but are not limited to nucleotide sequences comprising all or portions of TLR2 genes which are altered by the substitution of different codons that encode a functionally equivalent amino acid residue within the sequence, thus producing a silent change. By functionally equivalent amino acid residue, it must be understood, for example, an amino acid which belongs to the same class as the wild amino acid. The one skilled in the art knows the amino acid classes, which are the nonpolar (hydrophobic) amino acids, the polar neutral amino acids, the positively charged (basic) amino acids and the negatively charged (acidic) amino acids. Moreover, the TLR2 gene sequences used in the present invention can be produced by various methods known in the art.
Moreover, the one skilled in the art will be able to obtain an encoding nucleic acid for TLR2 using the mRNA of TLR2 obtained in Genbank under the numbers (NM_003264 for human and NM_011905 for mouse) and using reverse transcription.
The vector which can be used in the scope of the present invention to transform cells over-expressing TLR2 or TLR2 in association with TLR1 or TLR6 or others TLRs comprises at least one molecule of encoding nucleic acid for TLR2 as disclosed *supra,* advantageously associated with adapted control sequences, together with a production or expression process in a cellular host of TLR2. The vector used is chosen in function of the host (cultured cells) into which it is to be transferred; it can be any vector such as a plasmid. The preparation of these vectors as well as the production or expression in a protein host of the invention can be carried out by molecular biology and genetic engineering techniques well known to the one skilled in the art.

In addition, as it has been demonstrated that TLR2 must dimerize with others TLRs as TLR1 or TLR6 or others TLRs to detect ligands and induce signalling (Ozinsky et al., 2000, PNAS, vol. 97, pages 13766-13771; Takeuchi et al., 2002, J. Immunol. vol. 169, pages 10-14), the cells which express or which over-express TLR2 can also over-express other TLRs as TLR1 or TLR6. All what is discussed *supra* concerning TLR2 (i.e. molecule of encoding nucleic acid, vector) applies to others TLRs and more particularly to TLR1 and TLR6. The one skilled in the art will be able to obtain a molecule encoding for TLR1 or for TLR6 in Genbank respectively under the numbers NM_003263 for human TLR1 and NM_030682 for mouse TLR1; NM_006068 for human TLR6 and NM_011604 for mouse TLR6).

A compound, which increases the basal activity of a receptor, is normally called an agonist. Attachment of an agonist to a receptor brings about a change in the conformation of the receptor, and inside the cell, and this signal is transduced through the intermediary of second messengers. Consequently, the aim of step (b) in the process of the invention is, after putting in contact the compound capable of constituting agonist of the TLR2 or TLR2 in association with TLR1 or TLR6 or others TLRs, measuring by any appropriate means the affinity between said compound and said receptor.
The contact between the compound to be tested and the TLR2 or TLR2 in association with TLR1 or TLR6 or others TLRs can be carried out by using the cells expressing said receptors at least at their surface confirmed by antibody labelling and flow cytometry. If the compound tested constitutes an agonist, its contact with the transformed cells induces intracellular signals, which result from the fixation of said compound on the receptor.

Thus, the determination that the biologically active compound tested is a TLR2 agonist in step (b) of the method object of the present invention can be done by any method known by one skilled in the art. Advantageously, an increase in the basal activity level of TLR2 or TLR2 in association with TLR1 or TLR6 or others TLRs can be measured. Said increase in the basal activity can be measured thanks to the increase in the TLR2 induced activation. The TLR2 induced activation can be measured through activation of a signal transduction event (typically resulting in activation of NF-κB) or transcriptional activation of a reporter gene (typically regulated via NF-κB responsive elements).
The one skilled in the art will be able to find processes and conditions to measure activation of a signal transduction event such as serine/threonine kinase IRAK activity and NF-κB activation/translocation.
More particularly, the TLR2 induced activation can be measured by reporter gene assays as described in Werts et al. (2001, Nature Immunology, vol. 2, pages 346-352). The TLR2 induced activation measured is advantageously the NF-κB activation. TLR2 induced activation of a reporter gene such as alkaline phosphatase; luciferase or green fluorescent protein can be easily realised using a suitable colorimetric or fluorimetric assay. The one skilled in the art will find adequate conditions to perform these reporter gene assays.

In a second embodiment, the steps (a) and (b) of the method of the present invention can also be carried out by fixing one or several TLR2 on one or several membranes. The TLR2 can thus also be integrated with a biosensor. In such a system, it is possible to visualise in real time the interactions between the compound being tested and the receptor. One of the partners of the couple receptor/ligand is fixed on an interface, which can contain a matrix, covered with aliphatic chains. This hydrophobic matrix can easily be covered with a lipidic layer by spontaneous fusion of liposomes injected into its contact. The TLR2 inserted in the liposomes or vesicles can thus be integrated into the biosensors. The biologically active compound is thus analysed with regard to one or several TLR2. In this kind of experimentation, as discussed supra, the TLR2 can be in association with TLR1 or TLR6 or others TLRs.
Alternative approaches such as the agonist binding to human TLR2 will be tested using BioSensor, which allow in addition to measure binding affinity.

The present invention also concerns the use of any TLR2 agonist for the preparation of a medicine useful for the treatment and/or the prevention of asthma and/or allergies,
wherein the TLR2 agonist is chosen among the group constituted by *Treponema* glycolipid, *Trypanosoma cruzi* glycophosphatidylinositol anchor, lipoproteins derived from *Borrelia burgdorfei, Treponema pallidium* and *Mycoplasma fermentans* and leptospiral lipopolysaccharides.

The amount of the biologically active compound or TLR2 agonist which will be effective in the treatment and/or the prevention of asthma and/or allergies will depend on the nature of the disorder or condition and can be determined by standard experimental and clinical techniques. In addition, *in vitro* assays may optionally be employed to help identify optimal dosage ranges. Moreover, the precise dose to be employed in the formulation will also depend on the route of administration. Effective doses may be extrapolated from dose-response curves derived from *in vitro* or animal model test systems.

Other advantages and characteristics of the invention will become apparent by reading the following examples concerning the role of TLR2 in antigen induced bronchial hyperreactivity (BHR), and the inhibition by lipoprotein of allergic asthma, and in which:
Figure 1 concerns the increased BHR in antigen sensitised TLR2 KO versus wild-type mice;
Figures 2 concerns the increased in total number of cells into BAL (A) and recruitment of eosinophils into BAL of TLR2 KO mice (B);
Figure 3 concerns the increased peribronchial infiltration of eosinophils in TLR2 KO mice;
Figure 4 concerns the increased IL-4, IL-5 and IL-13 levels in BAL;
Figure 5 discloses increased specific serum IgE levels in TLR2 KO mice;
Figure 6 discloses inhibition by lipoprotein (LP, 10 ug) of BHR (A); eosinophil recruitment of eosinophil (B) in antigen sensitised wild-type mice.

### I. MATERIALS AND METHODS.

### I.1. Animals and immunisation.

The TLR2 -/- and +/+ mice were obtained from Dr C Kirschning. The local Ethics Committee approved all protocols employed in this study. The mice, aged 6 to 8 weeks, were immunised subcutaneously twice at weekly intervals with a 0.4 ml NaCl containing 100 g Ova and 1.6 mg alum. The mice were challenged intranasaly twice at weekly intervals, one week after the second immunisation, at days 14 and 21 under light i.v. ketamine anaesthesia (preventing coughing) by applying 2 x 25 µl Ova in alum-free saline solution (10 µg) or saline alone as a control. Bacterial lipoprotein (10 µg) was given 1 h prior to the challenge of Ova sensitised TLR2 +/+ mice.

### I.2. Airways resistance.

The airway resistance was evaluated by whole-body plethysmography 24Hr after the last challenge (day 22). Bronchial hyperreactivity to aerosolised methacholine was investigated at several time points after Ova challenge. Unrestrained conscious mice were placed in whole-body plethysmography chambers (Buxco Electronic, Sharon, CO, USA). Mice were ventilated to avoid hypoxemia induced by methacholine administration (Corry et al 1998). Methacholine at 300mM was aerosolised for 50 seconds and mean airway bronchoconstriction readings, as assessed by Enhanced Respiratory Pause (PenH), were obtained over 15-min periods. PenH can be conceptualised as the phase shift of the thoracic flow and the nasal flow curves; increased phase shift correlates with increased respiratory system resistance. PenH is calculated by the formula PenH = (Te/RT-1) x PEF/PIF, where Te is expiratory time, RT is relaxation time, PIF is maximal value of inspiratory flow, PEF is maximal value of expiratory flow.
PenH values correspond to mean of eleven events (cyclers) every five seconds in raw data.
After analysis of data, PenH values are indicated for 3 points (-3min to 1min) before and 14 points (+1min to +14min) after Methacholine nebulization. In this case, indicated PenH value at every point corresponds to the mean of PenH values between 1 min before and 1 min after the point (for example PenH values at +5 min correspond to the mean of all values between point +4min and point +6min).

### I.3. Bronchoalveolar lavage (BAL).

BAL was performed 3 days after intranasal challenge under strong ketamine anaesthesia by rinsing with 4 volumes of 0.5ml each of ice-cold PBS. The lavage fluid was centrifuged, resuspended, counted by a haematocytometer chamber and cytospin preparation were prepared using a Shandon cytocentrifuge. The cells were analysed after differential staining with May-Gruenwald-Giemsa.

### I.4. Lung histology.

After bronchoalveolar lavage, the mice were killed (3 days after Ova challenge). The whole lung was removed and fixed in 4% buffered formaldehyde for standard microscopic analysis using H&E and PAS (mucus) stains The peribronchial infiltrate and the smooth muscle hyperplasia was assessed by a semi-quantitative score (0 - 3) by two independent observers.

### I.5. Cytokine analysis.

BAL fluid was collected 48 Hr after the last challenge and analysed for IL-4, IL-5 and IL-13 by ELISA using commercial kits (Pharmingen).

### I.6. Evaluation of OVA specific serum IgE levels.

Blood was collected 48 Hr after the last challenge and serum prepared for OVA specific IgE dosage by ELISA using rat anti-mouse IgE (BD Biosciences) for capture and biotinylated ovalbumine and ExtrAvidine peroxidase conjugate for revelation. Sera from mice hyperimmunised with OVA in alum was used as standard for the OVA-specific IgE ELISA.

### I.7. Statistical analysis.

Data are presented as means and standard error of the mean (SEM) indicated by error bars. Statistical significance was determined using Student's *t* test. *P* values of <0.05 were considered statistically significant.

### II. RESULTS.

### II.1. TLR2 controls bronchial hyperreactivity and eosinophil recruitment.

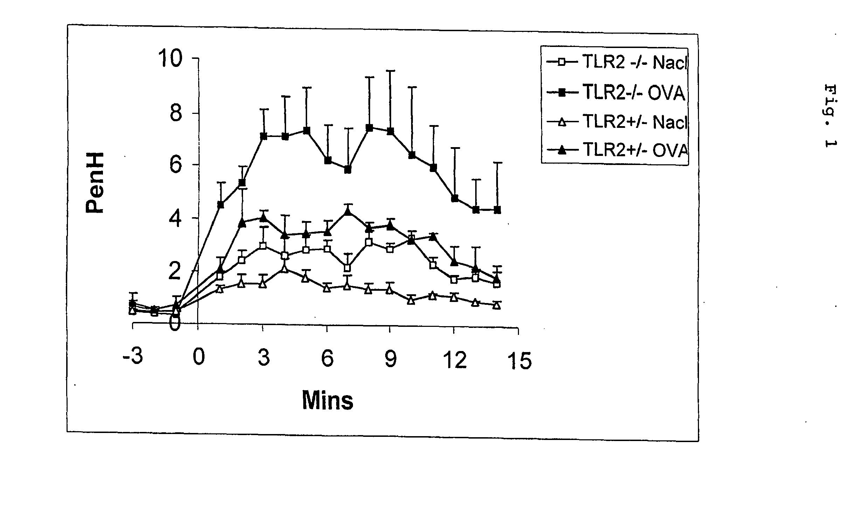

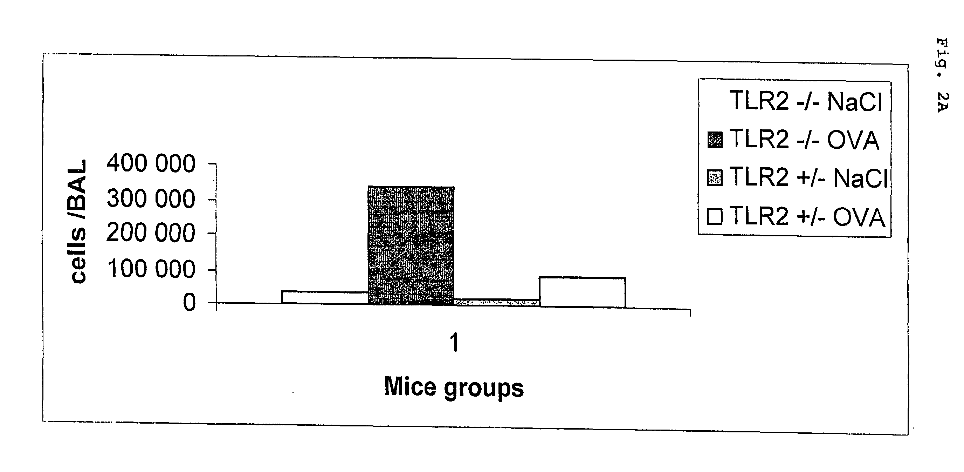

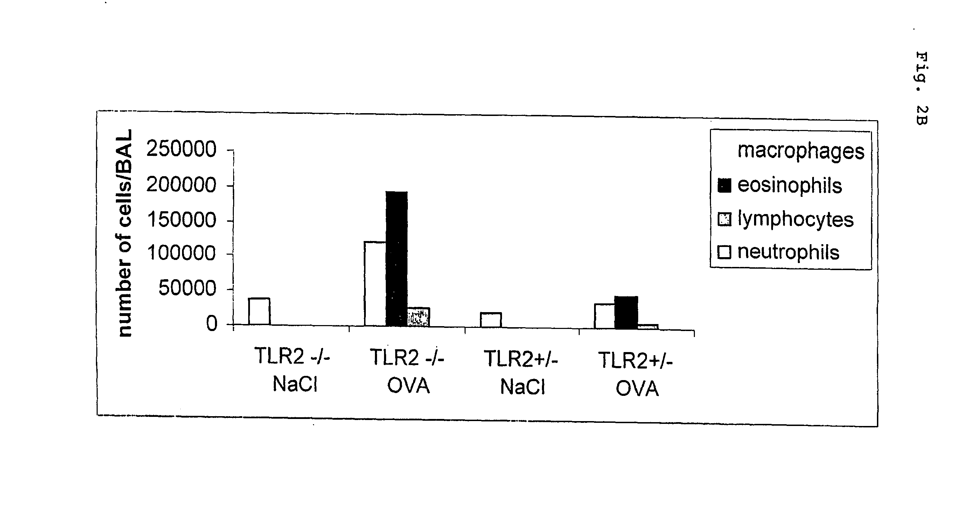

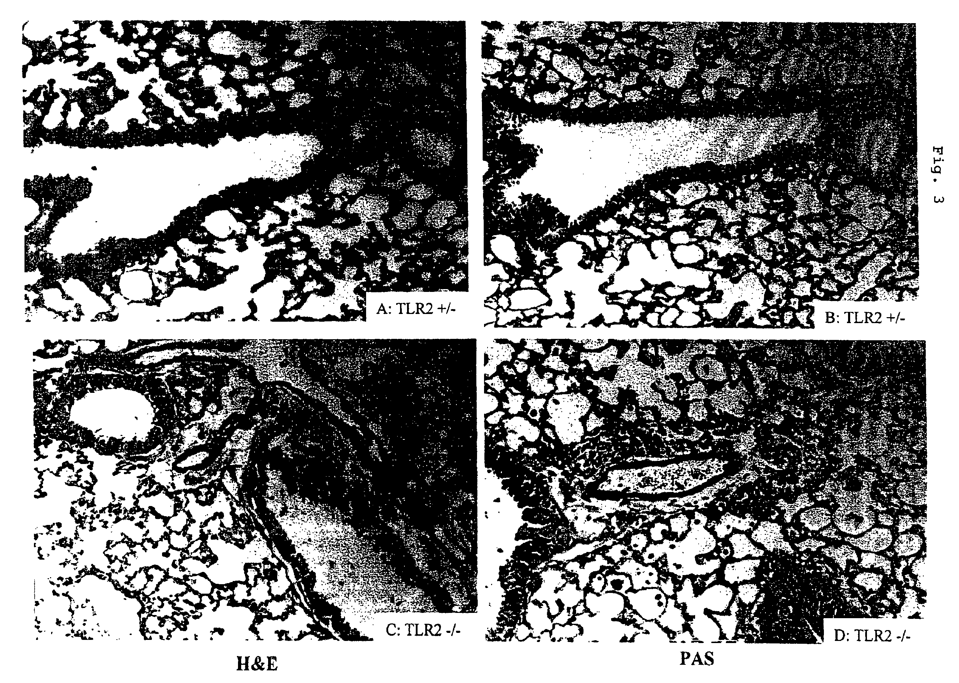

Firstly, the role of TLR2 was investigated in antigen (Ova) induced bronchial hyperreactivity (BHR). Antigen challenge in sensitised wild-type mice caused BHR (Figure 1). In the absence of TLR2, e.g. in TLR2 deficient mice, BHR was at least 2 fold increased as compared to sensitised wild-type controls (p<0.05).
Second, the total and differential cell counts were assessed in the BAL fluid in Ova sensitised mice. Antigen challenge of wild-type mice caused a distinct increase of mononuclear cells and especially eosinophils (Figures 2A and 2B). In TLR2 deficient mice a further significant increase of total cells and eosinophils in the BAL fluid was found (p<0.01). A marked recruitment of eosinophils could e demonstrated in lung sections from Ova sensitised and challenged mice, which was clearly more prominent in TLR2 deficient mice. The peribronchial cell recruitment is associated is with hyperplasia of the bronchial smooth muscle and goblet cells with mucus hypersecretion was more prominent in the absence of TLR2 as compared to controls (Figure 3).

### II.2. Increased IL-4, IL-5, IL-13 levels in BAL or serum IgE levels in TLR2 KO mice.

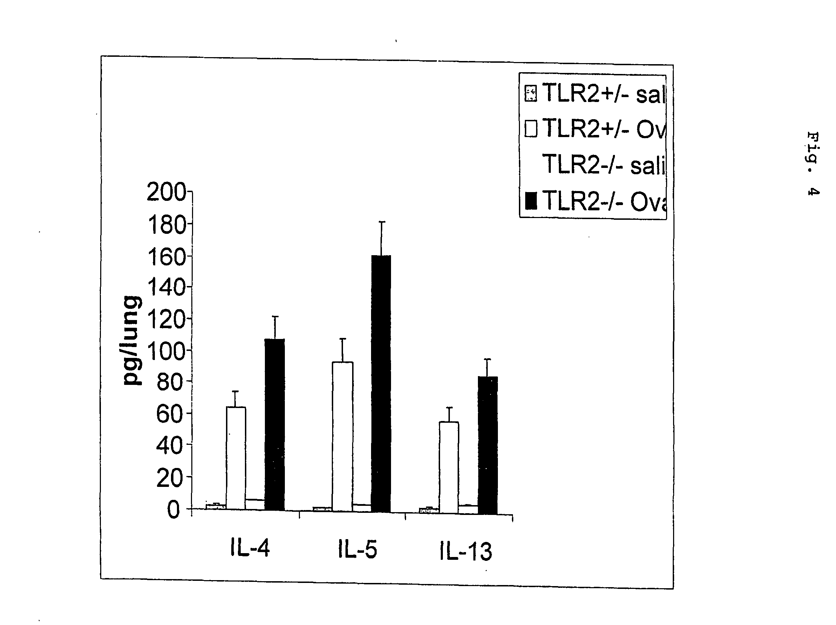

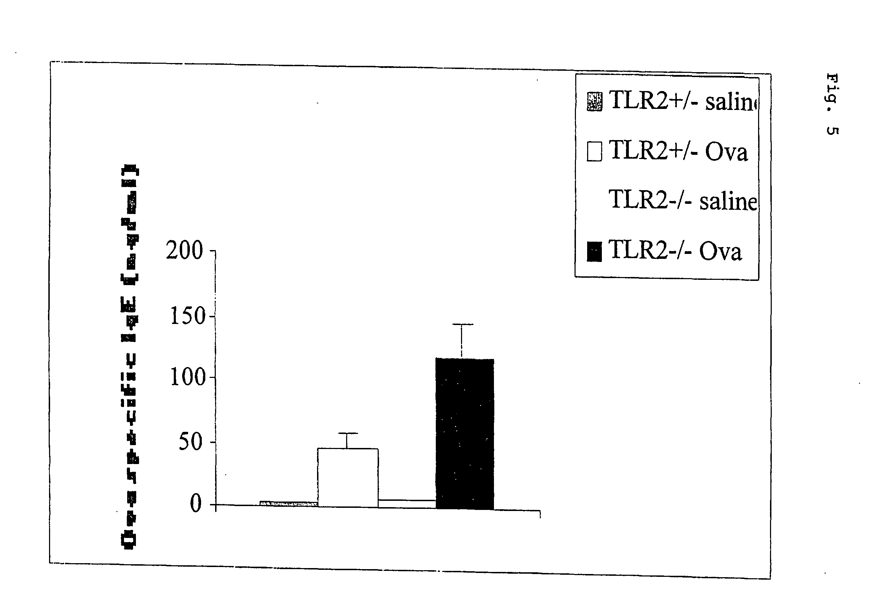

Allergic asthma is associated with an increased production of Th2 cytokines. Therefore, the inventors asked whether Th2 cytokines are enhanced in TLR2 KO mice. The levels of IL-4, IL-5 and IL-13 were increased in the BAL fluid of Ova challenged wild-type mice. Furthermore, in the absence of TLR2 the cytokine levels were significantly elevated (Figure 4).
The prevailing Th2 cytokine response is associated with increased IgE levels. Therefore, the increased cytokine levels would favor augmented IgE synthesis. Indeed, the IgE levels were further enhanced in the absence of TLR2 as compared to wild-type mice (Figure 5).
The present data suggest that TLR2 controls the allergic response, and absence of TLR2 enhances the production of Th2 cytokines and IgE production.

### II.3. Lipoprotein induces inhibition of allergic asthma.

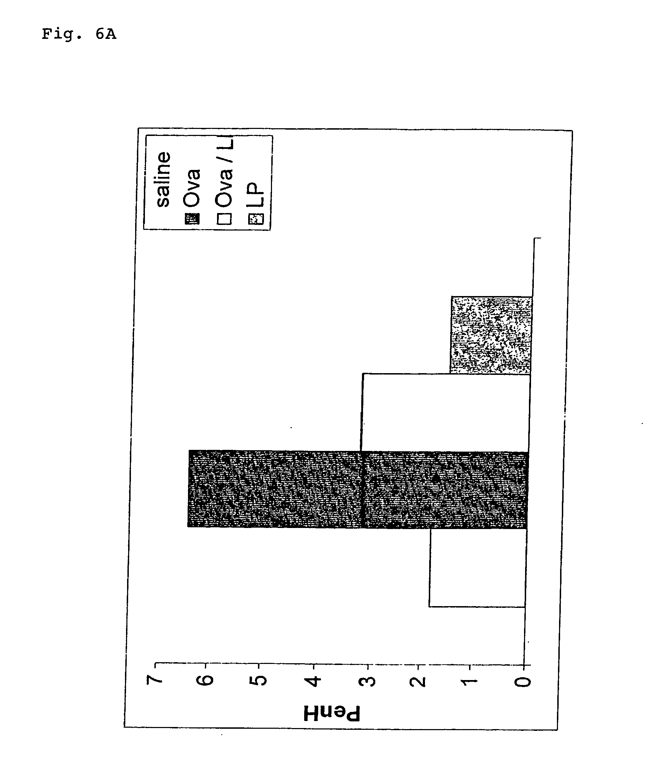

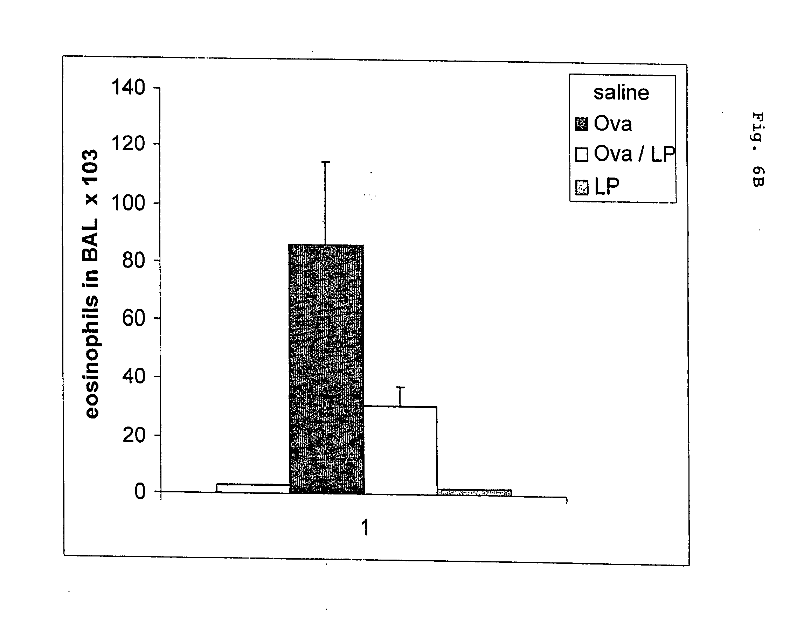

Since absence of TLR2 enhances the allergic response, the inventors tested the possibility that an agonist of TLR2 might modulate the BHR. The inventors administered the TLR2 agonist, lipoprotein (LP), intranasally at 10 ug just prior to the antigen challenge of sensitised wild-type mice.
The inventors show that LP prevented significantly BHR (Figure 6A). The inhibition of BHR by LP is comparable to that of the steroid budesonide (data not shown).
Furthermore, LP inhibited the cell recruitment into the BAL fluid, with a dramatic reduction of eosinophils in BAL (Figure 6B).
Together, the data obtained with the TLR2 agonist LP suggest that activation of TLR2 may inhibit allergic asthma in mice.

### III. DISCUSSION.

This study demonstrates for the first time that TLR2 recognition and signalling may be critical in the allergic bronchial response. TLR2 deficient mice have increased BHR, recruitment of eosinophils, IgE and IL-4 levels. Although the allergen, Ova, is not recognised by the innate immune receptor, environmental antigens including endotoxin and others may be recognised by TLR2 and signal a protective signal. If absent as in TLR2 deficient mice, a Th2 immune response prevails leading to enhanced asthma response.
Engagement of TLR2 on wild-type mice by the TLR2 agonist LP prevents BHR and the recruitment of eosinophils into the BAL and lung tissues.
In order to exploit these finding small chemical agonists of TLR2 need to be identified and tested in the prevention and treatment of allergic asthma.
The present study suggests that TLR2 represents a novel drug target of medicines to treat bronchial asthma and allergic disorders in general.

## Claims

1. Method for identifying a biologically active compound with anti-asthmatic, and/or anti-allergic properties wherein it comprises the following steps:
a) Contacting a TLR2 (Toll - like receptor 2) with said biologically active compound
b) Determining if said biologically active compound increases the basal activity of that said TLR2.

2. Method according to claim 1, wherein said step (a) comprises the following steps:
i) Culturing cells which express functional TLR2, and
ii) Incubating said cells with said biologically active compound.

3. Method according to claim 2, wherein the cells cultured in the step (i) are cells which over-express TLR2.

4. Method according to any one of claims 2 or 3, wherein the cells cultured in the step (i) are mammalian cells.

5. Method according to claim 4, wherein the mammalian cells are chosen in the group constituted by HEK293 and BaF3 cells.

6. Method according to any one of claims 2 to 5, wherein the cells cultured in the step (i) are transformed with a molecule of encoding nucleic acid for TLR2, or with a vector comprising at least one molecule of encoding nucleic acid for TLR2, advantageously associated with adapted control sequences.

7. Method according to claim 6, wherein the molecule of encoding nucleic acid for TLR2 is a molecule encoding for a mammalian TLR2 and, more preferably, a molecule encoding for a human TLR2.

8. Method according to any one of claims 2 to 7, wherein the cells cultured in the step (i) further over-express other TLRs such as TLR1 or TLR6.

9. Method according to any one of preceding claims, wherein said increase in the basal activity level of TLR2 is measured thanks to the increase in the TLR2 induced activation.

10. Method according to claim 9, wherein said TLR2 induced activation is measured through activation of a signal transduction event or transcriptional activation of a reporter gene.

11. Method according to claim 1, wherein, at said step (a), the TLR2 is inserted in liposomes or vesicles and integrated into bio-sensors.

12. Method according to claim 11, wherein TLR2 is in association with TLR1 or TLR6 or others TLRs.

13. Use of a TLR2 agonist for the preparation of a medicine useful for the treatment or the prevention of asthma and/or allergies, wherein said TLR2 agonist is chosen among the group constituted by *Treponema* glycolipid, *Trypanosoma cruzi* glycophosphatidylinositol anchor, lipoproteins derived from *Borrelia burgdorfei, Treponema pallidium* and *Mycoplasma fermentans,* and leptospiral lipopolysaccharides.

## Patentansprüche

1. Verfahren zum Identifizieren einer biologisch aktiven Verbindung mit Asthma lindernden und/oder gegen Allergie gerichteten Eigenschaften, wobei es die folgenden Schritte umfasst:
a) das Inkontaktbringen eines TLR2 (Toll-like Rezeptor 2) mit der genannten biologisch aktiven Verbindung,
b) das Bestimmen, der möglichen Steigerung der Basalaktivität des genannten TLR2 durch die biologisch aktive Verbindung.

2. Verfahren nach Anspruch 1, wobei der Schritt (a) die folgenden Schritte umfasst:
i) das Kultivieren von Zellen, die funktionellen TLR2 exprimieren, und
ii) das Inkubieren der Zellen mit der genannten biologisch aktiven Verbindung.

3. Verfahren nach Anspruch 2, wobei die Zellen, die in dem Schritt (i) kultiviert werden, Zellen sind, die TLR2 überexprimieren.

4. Verfahren nach einem der Ansprüche 2 oder 3, wobei die Zellen, die in dem Schritt (i) kultiviert werden, Säugerzellen sind.

5. Verfahren nach Anspruch 4, wobei die Säugerzellen aus der Gruppe ausgewählt sind, die aus HEK293- und BaF3-Zellen besteht.

6. Verfahren nach einem der Ansprüche 2 bis 5, wobei die Zellen, die in dem Schritt (i) kultiviert werden, mit einem Nukleinsäuremolekül, das für TLR2 kodiert, oder mit einem Vektor, der mindestens ein Nukleinsäuremolekül, das für TLR2 kodiert, umfasst, vorzugsweise mit angepassten Kontrollsequenzen assoziiert, transformiert werden.

7. Verfahren nach Anspruch 6, wobei das Nukleinsäuremolekül, das für TLR2 kodiert, ein Molekül ist, das für einen Säuger-TLR2 kodiert, und mehr bevorzugt ein Molekül, dass für einen humanen TLR2 kodiert.

8. Verfahren nach einem der Ansprüche 2 bis 7, wobei die Zellen, die in dem Schritt (i) kultiviert werden, andere TLR, wie TLR1 oder TLR6, überexprimieren.

9. Verfahren nach einem der vorhergehenden Ansprüche, wobei der genannte Anstieg des Basalaktivitätsniveaus von TLR2 aufgrund des Anstiegs der von TLR2 induzierten Aktivierung gemessen wird.

10. Verfahren nach Anspruch 9, wobei die von TLR2 induzierte Aktivierung durch Aktivierung eines Signaltransduktionsereignisses oder Transkriptionsaktivierung eines Reportergens gemessen wird.

11. Verfahren nach Anspruch 1, wobei in dem Schritt (a) das TLR2 in Liposome oder Vesikel eingeführt und in Biosensoren integriert wird.

12. Verfahren nach Anspruch 11, wobei TLR2 mit TLR1 oder TLR6 oder anderen TLR assoziiert ist.

13. Verwendung eines TLR2-Agonisten zur Herstellung eines Arzneimittels, das zur Behandlung oder Vorbeugung von Asthma und/oder Allergien geeignet ist, wobei der genannte TLR2-Agonist aus der Gruppe ausgewählt ist, die aus *Treponema-*Glycolipid, *Trypanosoma-cruzi*-Glycophosphatidylinositol-Anker, Lipoproteinen, die von *Borrelia burgdorfei, Treponema pallidium* und *Mycoplasma fermentans* abgeleitet sind, und Leptospiren-Lipopolysacchariden besteht.

## Revendications

1. Procédé d'identification d'un composé biologiquement actif ayant des propriétés anti-asthmatiques et/ou anti-allergiques, ledit procédé comprenant les étapes suivantes :
a) mettre en contact un TLR2 (récepteur Toll-like 2) avec ledit composé biologiquement actif,
b) déterminer si ledit composé biologiquement actif accroît l'activité de base dudit TLR2.

2. Procédé selon la revendication 1, dans lequel ladite étape (a) comprend les étapes suivantes :
i) cultiver des cellules qui expriment le TLR2 fonctionnel, et
ii) incuber lesdites cellules avec ledit composé biologiquement actif.

3. Procédé selon la revendication 2, dans lequel les cellules cultivées à l'étape (i) sont des cellules qui sur-expriment TLR2.

4. Procédé selon l'une quelconque des revendications 2 ou 3, dans lequel les cellules cultivées à l'étape (i) sont des cellules de mammifère.

5. Procédé selon la revendication 4, dans lequel les cellules de mammifère sont choisies dans le groupe constitué par les cellules HEK293 et BaF3.

6. Procédé selon l'une quelconque des revendications 2 à 5, dans lequel les cellules cultivées à l'étape (i) sont transformées avec une molécule d'acide nucléique codant pour TLR2, ou avec un vecteur comprenant au moins une molécule d'acide nucléique codant pour TLR2, avantageusement associée avec des séquences de contrôle adaptées.

7. Procédé selon la revendication 6, dans lequel la molécule d'acide nucléique codant pour TLR2 est une molécule codant pour un TLR2 de mammifère, et, de préférence, une molécule codant pour un TLR2 humain.

8. Procédé selon l'une quelconque des revendications 2 à 7, dans lequel les cellules cultivées à l'étape (i) sur-expriment en outre d'autres TLR tels que TLR1 ou TLR6.

9. Procédé selon l'une quelconque des revendications précédentes, dans lequel ledit accroissement du niveau de l'activité de base de TLR2 est mesuré grâce à l'accroissement de l'activation induite par TLR2.

10. Procédé selon la revendication 9, dans lequel ladite activation induite par TLR2 est mesurée par l'activation d'un évènement de transduction de signal ou l'activation transcriptionelle d'un gène rapporteur.

11. Procédé selon la revendication 1, dans lequel, lors de ladite étape (a), le TLR2 est inséré dans des liposomes ou des vésicules et intégré dans des bio-capteurs.

12. Procédé selon la revendication 11, dans lequel TLR2 est en association avec TLR1 ou TLR6 ou d'autres TLR.

13. Utilisation d'un agoniste de TLR2 pour la préparation d'un médicament utile pour le traitement ou la prévention de l'asthme et/ou d'allergies, dans laquelle ledit agoniste de TLR2 est choisi dans le groupe constitué par le glycolipide *Treponema,* l'ancrage de glycophosphatidylinositol *Trypanosoma cruzi,* les lipoprotéines dérivées de Borrelia burgdorfei, Treponema pallidium et *Mycoplasma fermentans,* et les lipopolysaccharides *leptospiral.*
